# EUROPEAN PATENT APPLICATION

(11) **EP 1 652 935 A1**
(43) Date of publication of application: **03.05.2006**
(21) Application number: 04077978.7
(22) Date of filing: 29.10.2004
(51) Int. Cl.: C12Q 1/68

(54) **Methods using mitochondrial nucleic acid for determining a health status of an individual**

(71) Applicant: PrimaGen Holding B.V., 1105 BA Amsterdam (NL); UMC Utrecht Holding B.V., 3584 CK Utrecht (NL)
(72) Inventor: Penning, Maarten Tjerk, 3737 ME Groenekan (NL); Maas, Henriette Catharina Gerarda Irene Martina, 1107 VK Amsterdam (NL); de Baar, Marinus Petrus, 1011 TB Amsterdam (NL); Voest, Emile Eugene, 3768 AL Soest (NL)
(74) Representative: Winckels, Johannes Hubertus F.

(57) **Abstract**

The invention describes means and methods for determining mitochondrion bound nucleic acid and describes the use thereof in diagnostics. Preferable cell free mitochondrion bound nucleic acid is determined.

### Annex to the application documents - subsequently filed sequences listing

SEQUENCE LISTING

<110>  PrimaGen Holding B.V.
       UMC Utrecht Holding B.V.

<120>  Mitochondrion bound nucleic acid for determining a health status
       of an individual

<130>  P71161EP00

<140>  04077978.7
<141>  2004-10-29

<160>  9

<170>  PatentIn version 3.2

<210>  1
<211>  49
<212>  DNA
<213>  Artificial

<220>
<223>  U1A P1


<220>
<221>  misc_feature
<222>  (1)..(49)

<400>  1
aattctaata cgactcacta tagggagagg cccggcatgt ggtgcataa                49


<210>  2
<211>  20
<212>  DNA
<213>  Artificial

<220>
<223>  U1A P2


<220>
<221>  misc_feature
<222>  (1)..(20)

<400>  2
tgcgcctctt tctgggtgtt                                                20


<210>  3
<211>  34
<212>  DNA
<213>  Artificial

<220>

<223>  U1A MB

<220>
<221>  misc_feature
<222>  (1)..(34)

<400>  3
cgcatgctgt aaccacgcac tctcctcgca tgcg                                    34

<210>  4
<211>  49
<212>  DNA
<213>  Artificial

<220>
<223>  mtRNA P1

<220>
<221>  misc_feature
<222>  (1)..(49)

<400>  4
aattctaata cgactcacta tagggagagg agacacctgc taggtgtaa                    49

<210>  5
<211>  22
<212>  DNA
<213>  Artificial

<220>
<223>  mtRNA P2

<220>
<221>  misc_feature
<222>  (1)..(22)

<400>  5
ggtgcccccg atatggcgtt cc                                                 22

<210>  6
<211>  30
<212>  DNA
<213>  Artificial

<220>
<223>  mtRNA MB

<220>
<221>  misc_feature
<222>  (1)..(30)

<400> 6
cgatccaagg acaaggcgtt cacaggatcg 30

<210> 7
<211> 49
<212> DNA
<213> Artificial

<220>
<223> mtDNA P1

<220>
<221> misc_feature
<222> (1)..(49)

<400> 7
aattctaata cgactcacta tagggaagaa ccgggctctg ccatcttaa 49

<210> 8
<211> 20
<212> DNA
<213> Artificial

<220>
<223> mtDNA P2

<220>
<221> misc_feature
<222> (1)..(20)

<400> 8
gtaatccagg tcggtttcta 20

<210> 9
<211> 34
<212> DNA
<213> Artificial

<220>
<223> mtDNA MB

<220>
<221> misc_feature
<222> (1)..(34)

<400> 9
cgtacgtgat atcatctcaa cttagtatcg tacg 34

SEQUENCE LISTING

<110> PrimaGen Holding B.V.
      UMC Utrecht Holding B.V.

<120> Mitochondrion bound nucleic acid for determining a health status
      of an individual

<130> P71161EP00

<140> 04077978.7
<141> 2004-10-29

<160> 9

<170> PatentIn version 3.2

<210> 1
<211> 49
<212> DNA
<213> Artificial

<220>
<223> U1A P1


<220>
<221> misc_feature
<222> (1)..(49)

<400> 1
aattctaata cgactcacta tagggagagg cccggcatgt ggtgcataa                49


<210> 2
<211> 20
<212> DNA
<213> Artificial

<220>
<223> U1A P2


<220>
<221> misc_feature
<222> (1)..(20)

<400> 2
tgcgcctctt tctgggtgtt                                                20


<210> 3
<211> 34
<212> DNA
<213> Artificial

<220>

<223>  U1A MB

<220>
<221>  misc_feature
<222>  (1)..(34)

<400>  3
cgcatgctgt aaccacgcac tctcctcgca tgcg                                    34

<210>  4
<211>  49
<212>  DNA
<213>  Artificial

<220>
<223>  mtRNA P1

<220>
<221>  misc_feature
<222>  (1)..(49)

<400>  4
aattctaata cgactcacta tagggagagg agacacctgc taggtgtaa                    49

<210>  5
<211>  22
<212>  DNA
<213>  Artificial

<220>
<223>  mtRNA P2

<220>
<221>  misc_feature
<222>  (1)..(22)

<400>  5
ggtgcccccg atatggcgtt cc                                                 22

<210>  6
<211>  30
<212>  DNA
<213>  Artificial

<220>
<223>  mtRNA MB

<220>
<221>  misc_feature
<222>  (1)..(30)

<400> 6
cgatccaagg acaaggcgtt cacaggatcg 30

<210> 7
<211> 49
<212> DNA
<213> Artificial

<220>
<223> mtDNA P1

<220>
<221> misc_feature
<222> (1)..(49)

<400> 7
aattctaata cgactcacta tagggaagaa ccgggctctg ccatcttaa 49

<210> 8
<211> 20
<212> DNA
<213> Artificial

<220>
<223> mtDNA P2

<220>
<221> misc_feature
<222> (1)..(20)

<400> 8
gtaatccagg tcggtttcta 20

<210> 9
<211> 34
<212> DNA
<213> Artificial

<220>
<223> mtDNA MB

<220>
<221> misc_feature
<222> (1)..(34)

<400> 9
cgtacgtgat atcatctcaa cttagtatcg tacg 34

## Description

The invention relates to the field of medicine and diagnostics therefore. The invention in particular relates to the diagnostics for determining a health status of an individual.

The field of diagnostics is rapidly expanding. With increasing knowledge of diseases more and more refined diagnostic tools are coming available. These diagnostics typically use specific characteristics of a disease. These specific characteristics are more often than not, genetic characteristics. These can be monitored on the protein level or on the nucleic acid level. Current trends are toward very precise diagnostics using specific genetic markers or antibodies or brute force methods using arrays of many different specific markers. These tests are very often designed to ever more finely discriminate between diseases and individuals. This increasing discrimination power often means that individuals are often screened several times with increasingly refined diagnostic tests. Methods of the present invention provide information at the top of this diagnostic cascade and are simple enough to incorporate in routine health screenings and check-ups. To this end the invention provides a method for determining a health status of an individual comprising determining mitochondrion bound nucleic acid in a sample of body fluid or faeces of said individual. Body fluid and faeces contains mitochondria. These mitochondria are ultimately destined to be destroyed. However, they are sufficiently intact to allow the determination of nucleic acid that is contained therein or associated therewith. With mitochondrion bound nucleic acid is therefore meant nucleic acid that is present in mitochondria or associated therewith. Mitochondrion bound nucleic acid is determined and the amount detected is indicative for the health status of the individual. Body fluid such as lymph fluid, blood, urine, brain fluid or saliva also contains cells which also contain mitochondria. In the present invention it is preferred that cell free mitochondrion bound nucleic acid is determined. Cell free in this context means that mitochondria that are associated with cells, either internal or linked thereto are not considered. This can be done in various ways. One way is to determine the amount of total mitochondrion bound nucleic acid and the amount of cellular mitochondrion bound nucleic acid in said sample and subtracting the amount of cellular mitochondrion bound nucleic acid from the amount of total mitochondrion bound nucleic acid. However, in samples that are rich in cells (such as blood) it is preferred that cellular mitochondria are essentially removed from the sample prior to determining cell free mitochondrion bound DNA. Removal is typically done by utilizing the difference in density and/or size of the mitochondrion. One such means is centrifugation leading to the collection of plasma or serum. However, more specific means such as antibody based separation methods are also possible. It is not necessary to quantitatively separate cells (cellular mitochondria) from mitochondria. With essentially free it is therefore meant for practical purposes free of cellular mitochondria. A sample that is made essentially mitochondria free has undergone at least one step wherein cells are preferentially removed from the sample. Methods for quantitating mitochondrial nucleic acid are described herein. Further methods and methods for quantitating mitochondrial nucleic acid in so-called duplex assay wherein two or more different nucleic acids are amplified simultaneously are described in WO 02/46470. For the US this application is incorporated by reference herein.

Both DNA and RNA can be determined in a method of the present invention. It is preferred that said nucleic acid comprises RNA. Although RNA is typically much less stable than DNA, it has been found that mitochondrion bound RNA correlates more precisely with the health status of the individual. Determining the health status preferably comprises determining whether said individual is suffering from cancer. It has been found that an elevated level of mitochondrion bound nucleic acid is correlated with individuals that suffer from cancer. This finding is not limited to certain types of cancers. All types of cancer analysed showed this correlation. In a preferred embodiment said cancer comprises ovarium, prostate, RCC, colorectal, ENT or lung cancer. Particularly when the body fluid was blood or plasma or serum. As blood, plasma or serum is also routinely collected from individuals that are tested for disease it is preferred that the sample comprises blood, and preferably plasma or serum. Particularly for prostate cancer, the body fluid is preferably urine.

Other parameters in the sample may also be determined apart from mitochondrion bound nucleic acid. This feature can be used to determine mitochondrion bound nucleic acid relative to another parameter in said sample. This can be used to standardize measurements. In a preferred embodiment, the other parameter comprises cell and mitochondrion free nucleic acid. As this nucleic acid is typically subject to the extracellular environment of the sample, it is preferred that this nucleic acid is DNA, as DNA is typically more stable than RNA in such environments. Other parameters can be already present in the sample or can be added (spiked) prior to the determining mitochondrion bound nucleic acid.

In a preferred embodiment mitochondrion bound nucleic acid in the sample is compared with a reference. The reference can be a value for a healthy individual(s) and/or a value for individuals having a particular health status, such a suffering from cancer.

As the amounts of mitochondrion bound nucleic acid in a cell free sample is typically not very high it is preferred that the method comprises a nucleic acid amplification step for amplifying mitochondrion bound nucleic acid. In a preferred embodiment the amplification step comprises NASBA. Such amplification steps can be adapted such that they can quantitate the target nucleic acid in the sample. Quantitation is therefore preferred in a method of the invention. With quantitation is meant that the amount of mitochondrion bound nucleic acid in the sample is determined. Although quantitation is preferred it is also possible to determine whether mitochondrion bound nucleic acid in the sample is higher or lower than a reference. In this case assessment of the exact amount (quantitation) is not necessary.

The sample may be fluid. However, the body fluid may also be present in the sample in dried form. In dried form the sample is more stable and does not require freezing or refrigeration. Thus in a preferred embodiment a sample of body fluid was dried and stored on a solid carrier prior to determining the amount of mitochondrion bound nucleic acid. In a preferred embodiment the solid carrier is paper. Suitable solid carriers such as paper are described in WO 03/080869. For the US this reference is incorporated by reference herein. This reference is also referred to for methods for storing and recovering nucleic acid from the solid carrier.

Cellular mitochondrion bound nucleic acid can also be another parameter. This parameter is tell tale for a number of different diseases of individuals. It is therefore preferred that the results of cell free mitochondrion bound nucleic acid and cellular mitochondrion bound nucleic acid are combined. As mentioned above, it is preferred that a method of the invention further comprises determining a non-mitochondrion bound nucleic acid in said sample. This can be used for instance for standardization purposes. Thus in one embodiment said non-mitochondrion bound nucleic acid is a control nucleic acid present in or provided to said sample.
In a preferred embodiment said control nucleic acid and mitochondrion bound nucleic acid is amplified in one amplification step. This so-called duplex analysis allows very accurate relative assessments of nucleic acid amounts.

The invention further provides a use of means for quantitating mitochondrion bound nucleic acid in a sample of body fluid of an individual for determining whether said individual is suffering from cancer. It further provides a use of means for quantitating mitochondrion bound nucleic acid in a sample of body fluid of an individual for predicting or following the effect of a cancer treatment in said individual suffering from cancer. The invention further provides a use of means for quantitating mitochondrion bound nucleic acid in a sample of body fluid of an individual for predicting or determining side effects of a treatment. Changes in cell free mitochondrion bound nucleic acid are early events and precede clinical manifestations of side effects.

### Examples

### Example 1

Plasma was isolated from blood of healthy donors and patients with progressive cancer, using a BD Vacutainer® CPT™ Cell Preparation Tube with Sodium Citrate, according to the manufacturers recommendations. The plasma samples were centrifuged at 3452 rcf for 15 minutes. The supernatant plasma was subsequently stored at -80°C. 100 µl of plasma was added to a 1.5 ml eppendorf tube containing 900 µl lysis buffer. The nucleic acid now present in the lysis buffer was further purified with the method described by Boom et al (1990). The isolated nucleic acid was eluted in 50 µl elution buffer.

In table 1 the primers and probes used in these examples are summarized. Standard NASBA nucleic acid amplification reactions were performed in a 20 µl reaction volume and contained: 40mM Tris-pH 8.5, 90mM KCI, 12mM MgCl2, 5mM dithiotreitol, 1mM dNTP's (each), 2mM rNTP's (each), 0.2µM primer P1, 0.2µM primer P2, 0.05µM molecular beacon, 375mM sorbitol, 0.105 µg/µl bovine serum albumin, 6.4 units AMV RT, 32 units T7 RNA polymerase, 0.08 units RNase H and input nucleic acid. For the amplification of RNA, the complete mixture, except the enzymes was, prior to adding the enzymes, heated to 65°C in order to denature any secondary structure in the RNA and to allow the primers to anneal. (In the case of DNA, 2 units of MSP II were added. The mix was incubated at 37°C for 15 minutes, followed by denaturation at 95°C.) After cooling the mixture to 41°C the enzymes were added. The amplification took place at 41°C for 90 min in a thermostated fluorimeter (CytoFluor 2000 or EasyQ Reader) and the fluorescent signal of the molecular beacon probe was measured every 45 seconds.
To achieve quantification, a dilution series of target sequence was amplified and the time points at which the reactions became positive (the time to positivity, TTP) were plotted against the input amounts of nucleic acid. This way a calibration curve was created that could be used to read TTP values of reactions with unknown amounts of input and deduce the input amount. All amplifications were performed in duplicate. The average of these duplicate reactions was considered as the value for this sample. If the difference between duplicate amplifications was < 0.5 log value, the value for this samples was considered "valid".

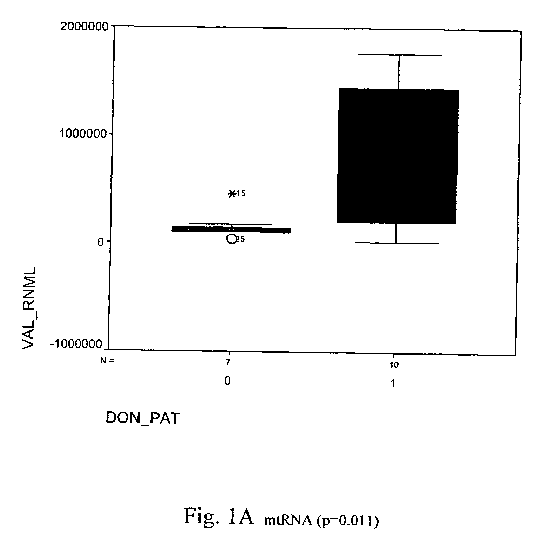

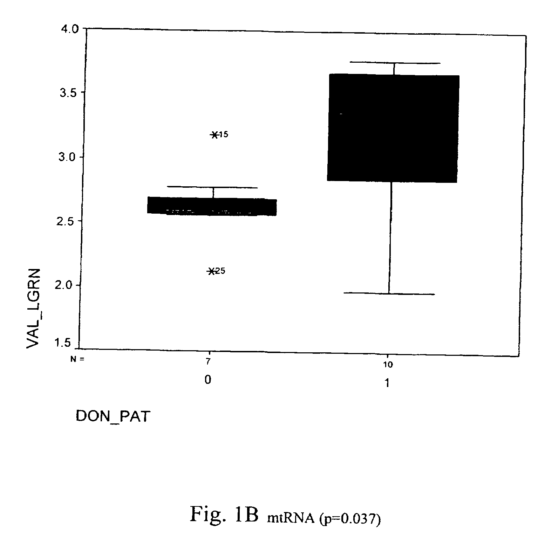

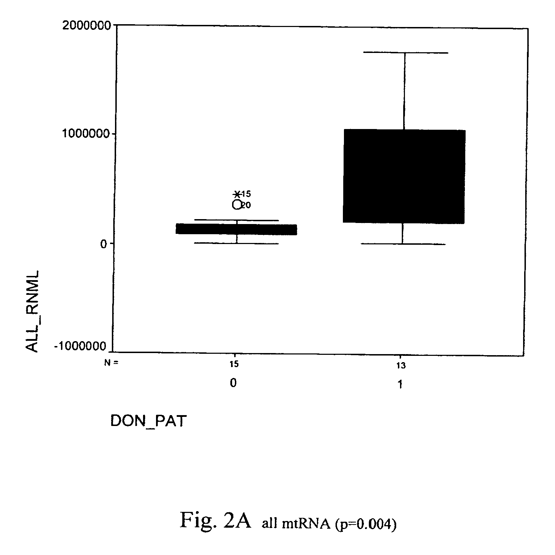

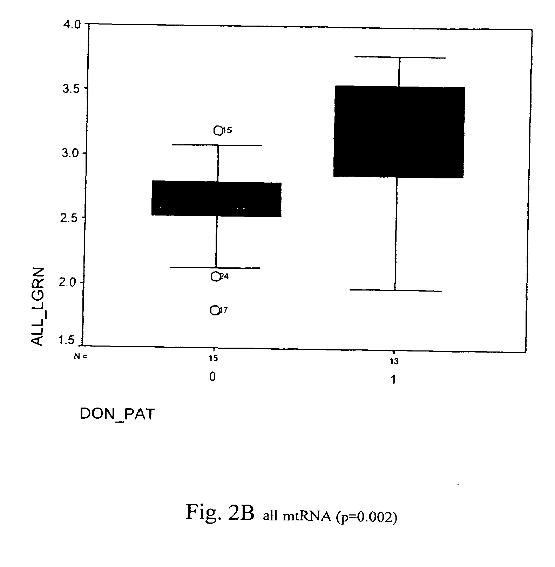

Analyzing the "valid" values, w find significantly higher mtRNA content in plasma from patients, compared to healthy donors (both when log values (fig. 1A) or linear values (fig. 1B) are compared). If we include the samples of which the difference between duplicates was more than 0.5 log, the significance became even stronger
(Figs. 2A and 2B), probably simply because more cases were included.

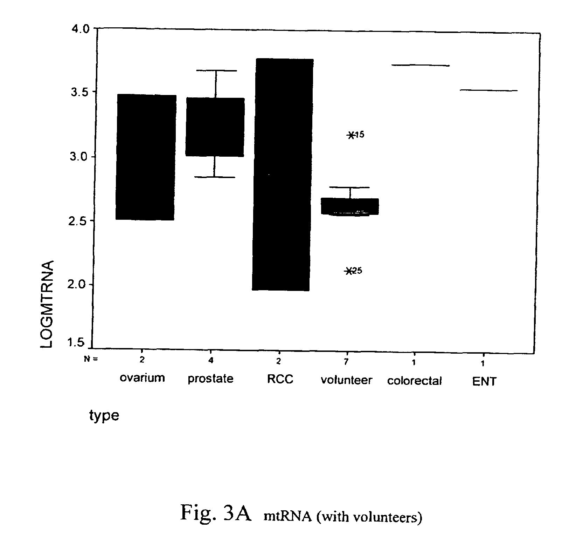

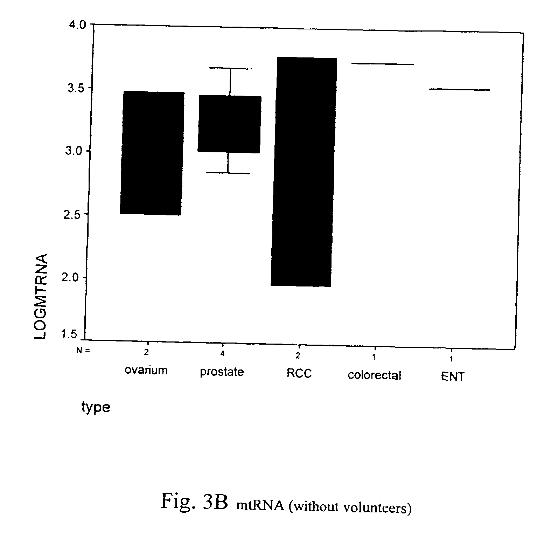

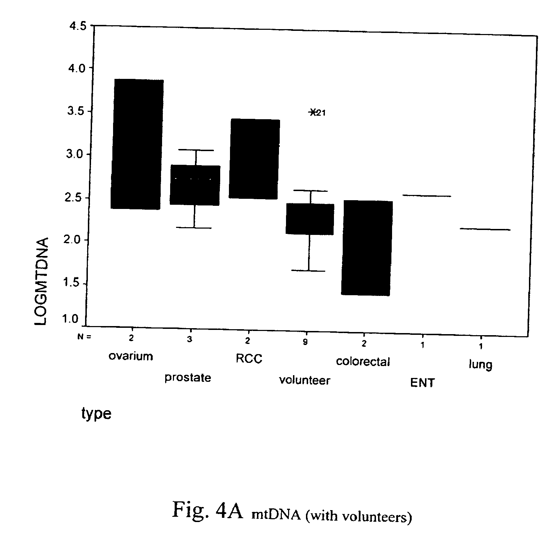

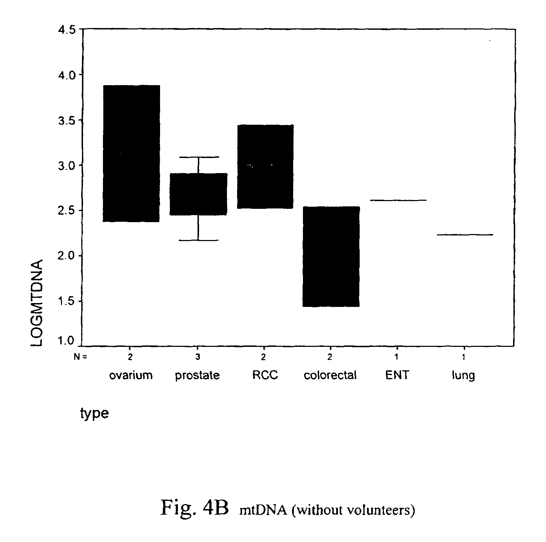

It is clear from Figures 1 and 2 that the variation in mtRNA is bigger in patients compared to healthy donors. Therefore we split the samples according to tumor type and looked at mtRNA (Fig. 3) and mtDNA (Fig. 4).

In spite of the small number of patients in each group, it is clear that there are differences in mtRNA and/or mtDNA content in the plasma of patients suffering from different tumors.

Mitochondrial DNA and RNA quantification in plasma.

### Example 2

Different labs use different protocols to isolate plasma from blood. There are a lot of different tubes on the market to separate cells from plasma, but some laboratories perform an additional centrifugation step after plasma is taken of the cells to get rid of residual cells or cell debris. In this example the effect of this additional centrifugation step on the quantification of mtDNA and mtRNA in plasma was investigated.

Plasma was isolated from the blood of two male healthy volunteers, using a BD Vacutainer® CPT™ Cell Preparation Tube with Sodium Citrate, according to the manufacturers recommendations (This involves centrifugation at 1692rcf). 300 µl of the plasma was aliquoted in eppendorf tubes and subjected to centrifugation at different speeds (0 rcf, 1700 rcf, 3400 rcf, 10000 rcf, and 16100 rcf). Two times 100 µl of centrifuged plasma was added to a 1.5 ml eppendorf tube containing 900 µl lysis buffer. The nucleic acid now present in the lysis buffer was further purified with the method described by Boom et al (1990). The isolated nucleic acid was eluted in 50 µl elution buffer.

In table 1 the primers and probes used in these examples are summarized. Standard NASBA nucleic acid amplification reactions were performed in a 20 µl reaction volume and contained: 40mM Tris-pH 8.5, 90mM KCl, 12mM MgC12, 5mM dithiotreitol, 1mM dNTP's (each), 2mM rNTP's (each), 0.2µM primer P1, 0.2µM primer P2, 0.05µM molecular beacon, 375mM sorbitol, 0.105 µg/µl bovine serum albumin, 6.4 units AMV RT, 32 units T7 RNA polymerase, 0.08 units RNase H and input nucleic acid. For the amplification of RNA, the complete mixture, except the enzymes was, prior to adding the enzymes, heated to 65°C in order to denature any secondary structure in the RNA and to allow the primers to anneal. (In the case of DNA, 2 units of MSP II were added. The mix was incubated at 37°C for 15 minutes, followed by denaturation at 95°C.) After cooling the mixture to 41°C the enzymes were added. The amplification took place at 41°C for 90 min in a thermostated fluorimeter (CytoFluor 2000 or EasyQ Reader) and the fluorescent signal of the molecular beacon probe was measured every 45 seconds.
To achieve quantification, a dilution series of target sequence was amplified and the time points at which the reactions became positive (the time to positivity, TTP) were plotted against the input amounts of nucleic acid. This way a calibration curve was created that could be used to read TTP values of reactions with unknown amounts of input and deduce the input amount. All amplifications were performed in duplicate. The average of these duplicate reactions was considered as the value for this sample.

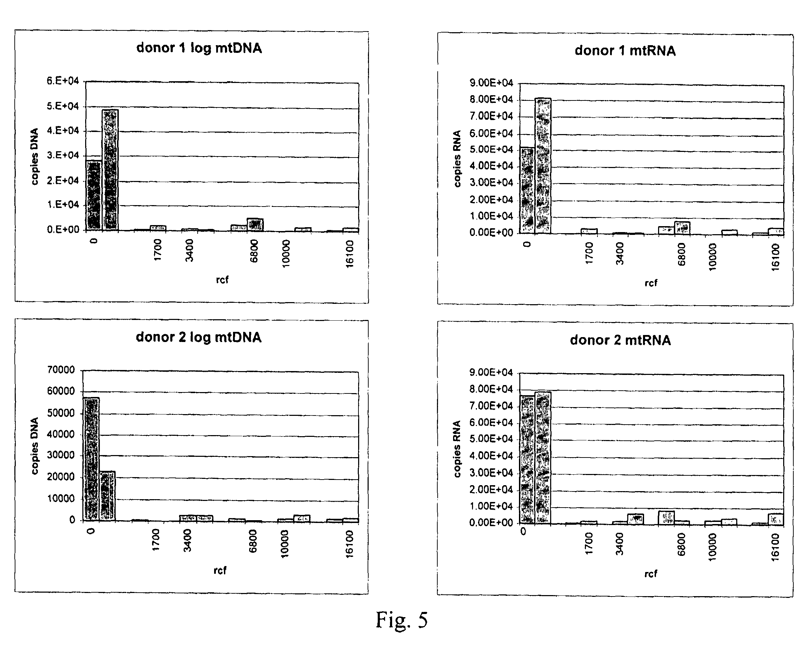

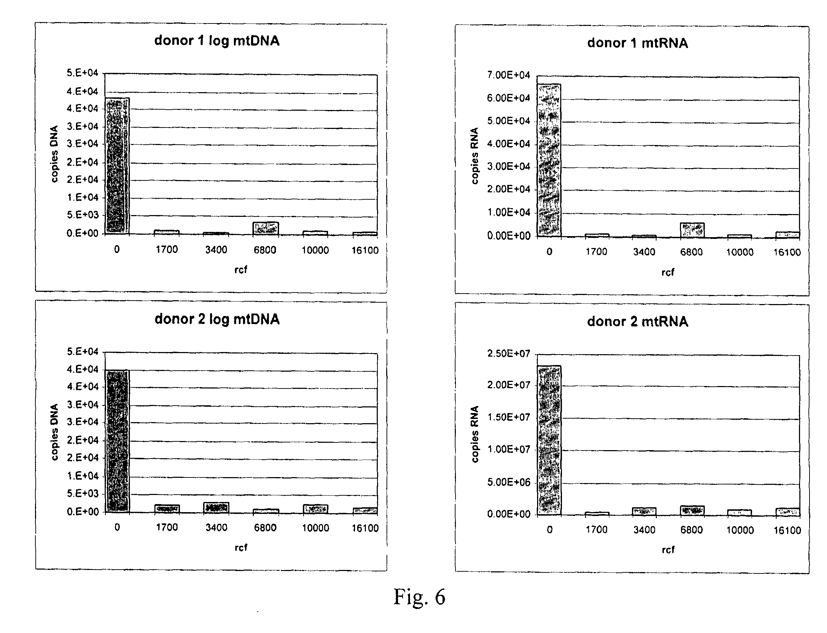

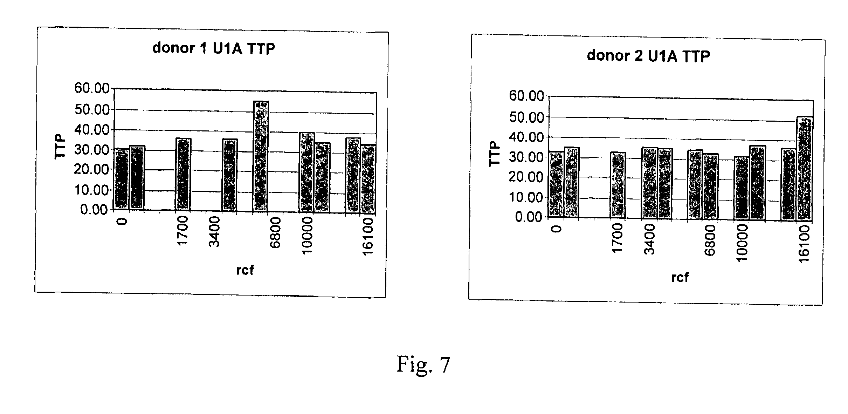

As shown in Figure 5 and 6, an additional centrifugation step influences the result of mtRNA and mtDNA quantification of plasma significantly. Surprisingly, centrifugation at 1700 rcf has the same effect as centrifugation at higher speeds. We would have expected to spin down cells at this lower speed and at higher speeds to spin down free circulating mitochondria. To further address this possibility, U1A was also studied. Although U1A copy numbers were lower than the quantification limit in all samples, TTP values were plotted (Figure 7). There seems to be no difference in the amount of U1A with or without centrifugation. This suggests that the plasma samples did not contain any residual cells.

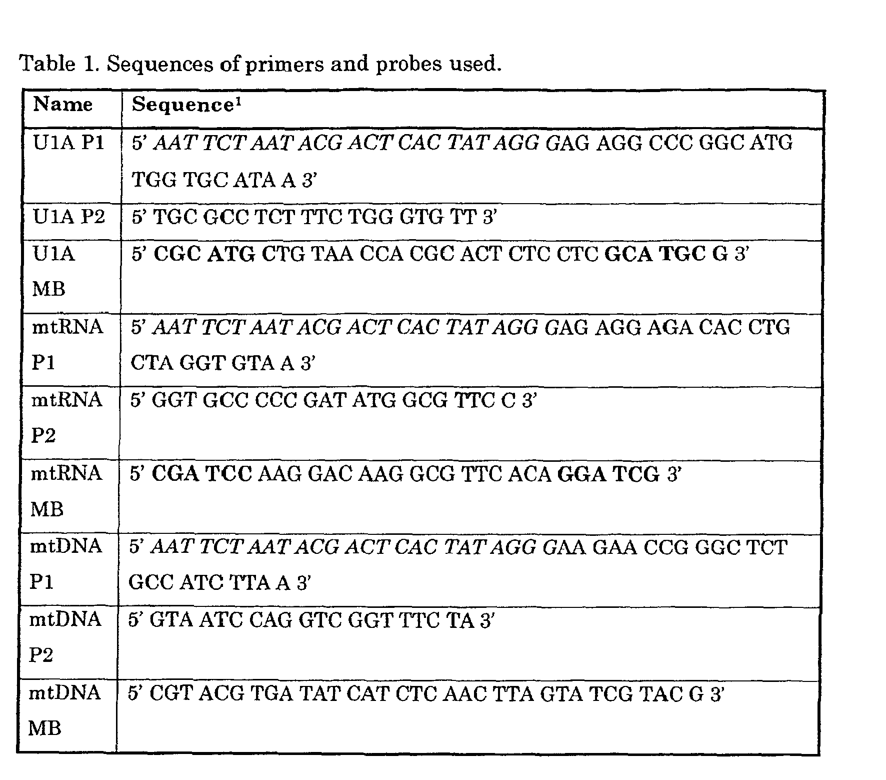

1. The T7 promoter part of primer P1 sequences is shown in *italics,* the stem sequences of the molecular beacon probes (MB) are shown in bold. The molecular beacon probes were labeled at the 3' end with DABCYL (the quencher) and at the 5' end with a fluorescent label, which is ROX in all cases.

## Claims

1. A method for determining a health status of an individual comprising determining mitochondrion bound nucleic acid in a sample of body fluid or faeces of said individual.

2. A method according to claim 1, comprising determining cell free mitochondrion bound nucleic acid.

3. A method according to claim 1 or claim 2, wherein the amount of cell free mitochondrion bound nucleic acid is determined by determining the amount of total mitochondrion bound nucleic acid and the amount of cellular mitochondrion bound nucleic acid in said sample and subtracting the amount of cellular mitochondrion bound nucleic acid from the amount of total mitochondrion bound nucleic acid.

4. A method according to any one of claims 1-3, wherein said nucleic acid comprises RNA.

5. A method according to any one of claims 1-4, wherein determining a health status comprises determining whether said individual is suffering from cancer.

6. A method according to any one of claims 1-5, wherein said sample comprises blood.

7. A method according to any one of claims 1-6, wherein said sample comprises plasma, serum or urine.

8. A method according to any one of claims 1-7, wherein said sample is essentially cell free.

9. A method according to any one of claims 1-8, wherein the mitochondrion bound nucleic acid is determined relative to another parameter in said sample.

10. A method according to any one of claims 1-9, wherein said amount is compared with a reference.

11. A method according to any one of claims 1-10, wherein determining the amount of mitochondrion bound nucleic acid comprises a nucleic acid amplification step.

12. A method according to claim 11, wherein said amplification step comprises NASBA.

13. A method according to claim 11 or claim 12, wherein nucleic acid amplified is quantitated.

14. A method according to any one of claims 1-13, wherein said sample of body fluid was dried and stored on a solid carrier prior to determining the amount of mitochondrion bound nucleic acid.

15. A method according to claim 14 wherein said solid carrier is paper.

16. A method according to any one of claims 1-15, further comprising determining cellular mitochondrion bound nucleic acid.

17. A method according to any one of claims 1-16, further comprising determining a non-mitochondrion bound nucleic acid in said sample.

18. A method according to claim 17, wherein said non-mitochondrion bound nucleic acid is a control nucleic acid present in or provided to said sample.

19. A method according to any one of claims 1-18, wherein said control nucleic acid and mitochondrion bound nucleic acid is amplified in one amplification step.

20. A method according to any one of claims 1-19, wherein a primer, a primer pair or a probe according to table 1 is used.

21. A method according to claim 20, wherein said primer, primer pair or probe is a U1A primer, primer pair or probe.

22. A method according to claim 20, wherein said primer, primer pair or probe is a mtRNA primer, primer pair or probe.

23. Use of means for quantitating mitochondrion bound nucleic acid in a sample of body fluid of an individual for determining whether said individual is suffering from cancer.

24. Use of means for quantitating mitochondrion bound nucleic acid in a sample of body fluid of an individual for predicting or following the effect of a cancer treatment in said individual suffering from cancer.

25. Use of means for quantitating mitochondrion bound nucleic acid in a sample of body fluid of an individual for predicting or determining side effects of a treatment.
